(19) Europäisches Patentamt
European Patent Office
Office européen des brevets

(11) Veröffentlichungsnummer: **0 166 331**
**A2**

# (12) EUROPÄISCHE PATENTANMELDUNG

(21) Anmeldenummer: **85107405.4**

(22) Anmeldetag: **15.06.85**

(51) Int. Cl.⁴: **C 07 D 413/12**, A 61 K 31/41

(30) Priorität: **26.06.84 DE 3423429**

(43) Veröffentlichungstag der Anmeldung: **02.01.86 Patentblatt 86/1**

(84) Benannte Vertragsstaaten: **AT BE CH DE FR GB IT LI NL SE**

(71) Anmelder: **Beiersdorf Aktiengesellschaft, Unnastrasse 48, D-2000 Hamburg 20 (DE)**

(72) Erfinder: **Cohnen, Erich, Dr., Moorende 74, D-2155 Jork (DE)**
Erfinder: **Jacobitz, Petra, Dr., Isestrasse 54, D-2000 Hamburg 13 (DE)**

## (54) Substituierte Phenoxyalkylaminopropanole, Verfahren zu ihrer Herstellung und ihre Verwendung sowie diese Verbindungen enthaltende Zubereitungen.

(57) Substituierte Phenoxyalkanolaminopropanole der allgemeinen Formel I

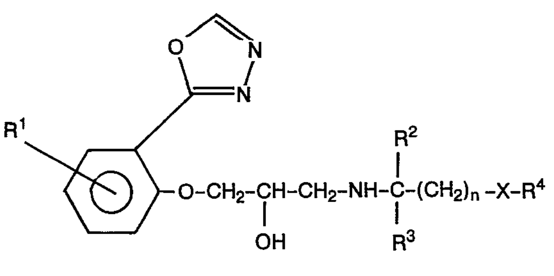

(I)

in der
$R^1$ ein Wasserstoff- oder Halogenatom, eine Alkyl- oder Alkoxygruppe mit jeweils 1 bis 4 Kohlenstoffatomen, eine Aminogruppe oder Acylaminogruppe,
$R^2$ und $R^3$, die gleich oder verschieden sein können, ein Wasserstoffatom oder eine Alkylgruppe mit 1 bis 3 Kohlenstoffatomen,
$R^4$ eine Phenylgruppe oder eine substituierte Phenylgruppe, die durch ein Halogenatom, eine oder zwei Alkoxygruppen, eine Methylendioxy-Gruppe, eine Hydroxy-Gruppe substituiert sein kann, eine Pyridylgruppe, eine Indolyl-Gruppe, eine gegebenenfalls durch ein Halogenatom substituierte 1,2-Benzisoxazolylgruppe, den Benzimidazol-2-on-, 1,4-Benzodioxan- oder Benzoxolan-Rest,
X ein Sauerstoffatom oder eine Einfachbindung und
n die Zahlen 0, 1, 2 oder 3 bedeuten,
sowie ihre Säureadditionssalze, blockieren sowohl die Alphaals auch die Betarezeptoren des adrenergen Systems und sind daher zur Behandlung von Hypertonie, Durchblutungsstörungen, Angina pectoris und Coronarinsuffizienz geeignet.

EP 0 166 331 A2

Beiersdorf Aktiengesellschaft
Hamburg

Substituierte Phenoxyalkylaminopropanole, Verfahren zu ihrer Herstellung und ihre Verwendung sowie diese Verbindungen enthaltende Zubereitungen

Gegenstand der Erfindung sind neue substituierte Phenoxyalkanolaminopropanole der allgemeinen Formel I

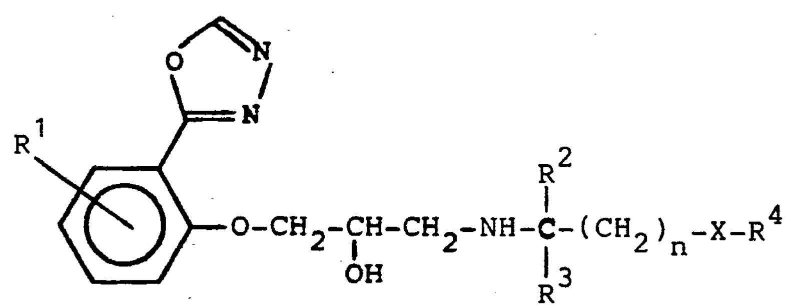

(I)

in der

$R^1$ ein Wasserstoff- oder Halogenatom, eine Alkyl- oder Alkoxygruppe mit jeweils 1 bis 4 Kohlenstoffatomen, eine Aminogruppe oder Acylaminogruppe,

$R^2$ und $R^3$, die gleich oder verschieden sein können, ein Wasserstoffatom oder eine Alkylgruppe mit 1 bis 3 Kohlenstoffatomen,

$R^4$ eine Phenylgruppe oder eine substituierte Phenylgruppe, die durch ein Halogenatom, eine oder zwei Alkoxygruppen, eine Methylendioxy-Gruppe, eine Hydroxy-Gruppe substituiert sein kann, eine Pyridylgruppe, eine Indolylgruppe, eine gegebenenfalls durch ein Halogenatom substituierte 1,2-Benzisoxazolylgruppe, den Benzimidazol-2-on-, 1,4-Benzodioxan- oder Benzoxolan-Rest,

X ein Sauerstoffatom oder eine Einfachbindung und

n die Zahlen 0, 1, 2 oder 3 bedeuten,

sowie ihre Säureadditionssalze, Verfahren zu ihrer Herstellung und ihre Verwendung und Zubereitungen, die diese Verbindungen enthalten.

Obgleich pharmazeutisch verträgliche Salze der neuen Verbindungen der Formel I bevorzugt sind, liegen alle Säureadditionssalze innerhalb des Bereichs der Erfindung. Alle Säureadditionssalze sind wertvoll zur Herstellung der Basen, selbst wenn das spezielle Salz nur als Zwischenprodukt gewünscht wird, wie z.B., wenn das Salz nur für Zwecke der Reinigung oder Identifizierung gebildet wird, oder wenn es als ein Zwischenprodukt bei der Herstellung eines pharmazeutisch verträglichen Salzes, wie z.B. durch Ionenaustauschverfahrensweisen, verwendet wird.

Die neuen Verbindungen der allgemeinen Formel I enthalten ein oder zwei asymetrische Kohlenstoffatome. Daher sind auch die verschiedenen optischen Isomeren sowie die Diastereoisomeren Gegenstand der Erfindung ebenso wie die Additionssalze dieser Verbindungen mit Säuren. Racemate können nach an sich bekannten Methoden in ihre optischen Antipoden aufgetrennt werden.

Die erfindungsgemäßen Alkylgruppen sowie die Alkylteile der Alkoxygruppen können geradkettig oder verzweigt sein und sind vorzugsweise Methyl-, Ethyl- und Propylgruppen.

Halogen ist Fluor, Chlor, Brom oder Jod, vorzugsweise Fluor, Chlor oder Brom, insbesondere aber Chlor.

Bevorzugte Acylgruppen sind Alkylcarbonylgruppen mit geradkettigen oder verzweigten Alkylgruppen und vorzugsweise 1 bis 10 Kohlenstoffatomen, insbesondere geradkettige Alkylcarbonylgruppen mit 1 bis 7 Kohlenstoffatomen. Besonders bevorzugt ist davon die Formyl-, Acetyl-, Propionyl- und Heptanoylgruppe.

$R^1$ ist vorzugsweise Halogen, insbesondere Chlor, oder die Acylaminogruppe. Halogensubstituenten $R^1$ befinden sich vorzugsweise in der 4-Stellung und die Acylaminogruppe vorzugsweise in der 5-Stellung des Phenylrings bezüglich des Oxadiazolrestes.

Die Reste $R^2$ und $R^3$ sind vorzugsweise Wasserstoff oder geradkettige Alkylgruppen, insbesondere Methyl und Ethyl.

Bedeutet X eine Einfachbindung, ist $R^4$ an die $-(CH_2)_n$-Gruppe beziehungsweise an das die Reste $R^2$ und $R^3$ tragende Kohlenstoffatom gebunden. Für n=O bedeutet $-(CH_2)-$ ebenfalls eine Einfachbindung, die Reste X oder $R^4$ sind dann mit dem die Reste $R^2$ und $R^3$ tragenden Kohlenstoffatom verbunden.

1,2-Benzisoxazolgruppen $R^4$ können ein Halogenatom tragen oder unsubstituiert sein.

Die folgenden Verbindungen der Formel I und deren Salze mit hohem therapeutischem Effekt werden besonders bevorzugt und zwar in der Form der Racemate sowie in der Form optisch aktiver Isomerer (Beispiel 4, 10, 6 u. 12):

2-[2-[2-Hydroxy-3-(3-phenyl-1,1-dimethyl-propylamino)-propoxy]-4-chlor-phenyl]-1,3,4-oxadiazol

2-[2-[2-Hydroxy-3-(2-(2-methoxy-phenoxy)-äthylamino)-propoxy]-4-chlor-phenyl]-1,3,4-oxadiazol

2-[2-[2-Hydroxy-3-(3-(2-methoxy-phenyl)-1,1-dimethyl-propylamino)-propxy]-4-chlor-phenyl]1,3,4-oxadiazol

2-[2-[2-(Hydroxy-3-(2-(2-methoxy-phenoxy)-äthylamino)-propoxy]-5-heptano ylamino-phenyl]1,3,4-oxadiazol

Die erfindungsgemäßen Verbindungen der Formel I und ihre physiologisch verträglichen Säureadditionssalze sind therapeutische Wirkstoffe, besitzen hohe pharmakologische Wirkung und sind wertvolle Arzneimittel. Sie blockieren sowohl die Alpharezeptoren als auch die Betarezeptoren des adrenergen Systems und sind daher vorzugsweise zur Behandlung von Hypertonie, Durchblutungsstörungen, insbesondere peripheren Durchblutungsstörungen, Angina pectoris und Coronarinsuffizienz geeignet.

Die Kombination einer alpharezeptorenblockierenden Eigenschaft mit einer betarezeptorenblockierenden ist aus zwei Gründen sinnvoll:

1. Im Gegensatz zu einer Betablockade allein wird der erhöhte Blutdruck vorwiegend durch die über Alpharezeptoren vermittelte Reduktion des peripheren Gefäßwiderstandes gesenkt; ein reflektorischer Anstieg des Herzzeitvolumens wird durch die betarezeptorenblockierende Eigenschaft der Substanzen verhindert.

2. Die Aktivierung des Renin-Angiotensinsystems, die nach Vasodilatation durch Alphablockade beobachtet wird und einen blutdrucksteigernden Einfluß ausübt, wird durch Betablockade unterdrückt.

Die in vorliegender Erfindung genannten Verbindungen senken bei Kaninchen in einer Dosis von 10-100mg/kg. p.o. die Isoprenalin-bedingte Tachycardie um 50% ($\beta_1$-Blockade) und den Phenylephrin-bedingten Blutdruckanstieg um 50% ($\alpha_1$-Blockade).

Die Verbindungen der vorliegenden Erfindung können oral oder parenteral angewendet werden. Die Einzeldosis beträgt beim Menschen 1 mg bis 500 mg, vorzugsweise 10 mg bis 100 mg, insbesondere 30 mg bis 50 mg. Diese Dosierungen sind vorteilhaft zur Behandlung der vorstehend genannten Krankheiten, insbesondere zur Behandlung von Hypertonie.

Die Tagesdosis ist, wie für Alpha- und Betarezeptoren üblich, individuell abzustimmen, weil sie von der Rezeptorenempfindlichkeit und dem Sympathikotonus des Patienten abhängt. Zweckmäßigerweise wird die Behandlung mit niederen Dosen begonnen und dann gesteigert.

Gemäß der Erfindung werden pharmazeutische Zusammensetzungen geschaffen, die eine Verbindung der Formel I oder deren pharmazeutisch verträgliche Salze, zusammen mit einem pharmazeutisch verträglichen Verdünnungsmittel oder Träger enthalten.

Die Verbindungen gemäß der Erfindung können mit üblichen pharmazeutisch verträglichen Verdünnungsmitteln oder Trägern und gegebenenfalls mit anderen Hilfsmitteln vermischt und beispielsweise oral oder parenteral verabreicht werden. Sie können oral in Form von Tabletten, Dragees, Sirups, Suspensionen und Flüssigkeiten oder parenteral in Form von Lösungen oder Suspensionen verabreicht werden. Oral zu verabreichende Präparate können ein oder mehrere Zusätze, wie Süßungsmittel, Aromatisierungsmittel, Farbstoffe und Konservierungsmittel, enthalten. Tabletten können den Wirkstoff mit üblichen, pharmazeutisch verträglichen Hilfsmitteln vermischt enthalten, zum Beispiel inerten Verdünnungsmitteln wie Calciumcarbonat, Natriumcarbonat, Lactose und Talk, Granulierungsmitteln und Mitteln, die den Zerfall der Tabletten bei oraler Verabreichung fördern, wie Stärke oder Alginsäure, Bindemitteln wie Stärke oder Gelatine, Gleitmitteln wie Magnesiumstearat, Stearinsäure und Talk.

Geeignete Trägerstoffe sind beispielsweise Milchzucker (Lactose), Gelatine, Maisstärke, Stearinsäure, Äthanol, Propylenglycol, Äther des Tetrahydrofurfuryl alkohols und Wasser.

Die Tabletten können nach bekannten Arbeitsweisen überzogen werden, um den Zerfall und die Resorption im Magen-Darmtrakt zu verzögern, wodurch die Aktivität des Wirkstoffs sich über eine längere Zeitspanne erstrecken kann. Ebenso kann in den Suspensionen der Wirkstoff mit Hilfsmitteln vermischt sein, die für die Herstellung solcher Zusammensetzungen üblich sind, zum Beispiel Suspendiermitteln wie Methylcellulose, Tragacanth oder Natriumalginat, Netzmitteln wie Lecithin, Polyoxyäthylen

stearat und Polyoxyäthylensorbitanmonooleat, und Konservierungsmitteln wie Äthylparahydroxybenzoat. Kapseln können den Wirkstoff als einzigen Bestandteil oder vermischt mit einem festen Verdünnungsmittel wie Calciumcarbonat, Calciumphosphat oder Kaolin enthalten. Die injizierbaren Präparate werden ebenfalls in an sich bekannter Weise formuliert. Die pharmazeutischen Präparate können den Wirkstoff in einer Menge von 0,1 bis 90%, insbesondere 1 bis 90%, enthalten, wobei der Rest ein Trägerstoff oder Zusatzstoff ist. Im Hinblick auf die Herstellung und Verabreichung werden feste Präparate, wie Tabletten und Kapseln, bevorzugt. Vorzugsweise enthalten die Präparate den Wirkstoff in einer Menge von 10 bis 50 mg.

Das Verfahren zur Herstellung der Verbindungen der Formel I ist dadurch gekennzeichnet, daß man Verbindungen der allgemeinen Formel IIa oder IIb oder deren Gemische,

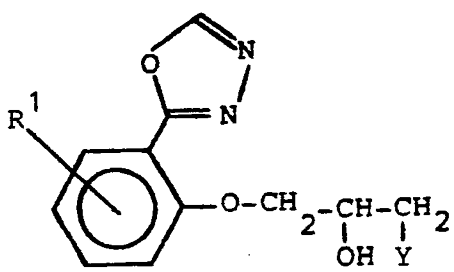

(IIa)

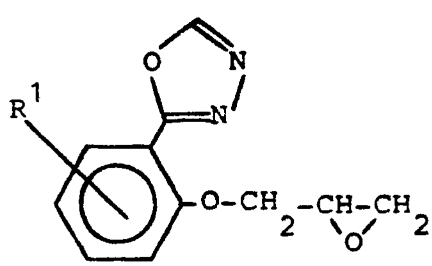

(IIb)

wobei Y für ein Halogenatom steht, mit einem Amin der allgemeinen Formel III

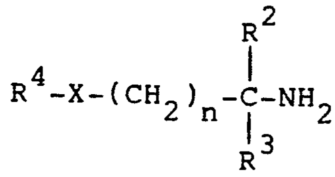

(III)

in der $R^2$, $R^3$, $R^4$, X und n die oben angegebene Bedeutung haben, umsetzt.

Die Reaktionen werden in einem Lösungsmittel, beispielsweise einem Alkohol, bei Temperaturen von 20 - 80°C durchgeführt. Die erhaltenen Verbindungen werden gegebenenfalls mit einer physiologisch verträglichen Säure in die Säureadditionssalze überführt.

Die Ausgangsverbindungen der Formeln IIa und IIb sind in der europäischen Patentschrift 45 955 und der deutschen Offenlegungsschrift 28 11 638 und in der Literatur beschrieben oder können analog zu bekannten Methoden erhalten werden. Auch die Amine der Formel III sind bekannte, in der Literatur beschriebene Verbindungen oder können nach Analogverfahren synthetisiert werden.

Die Verbindungen der allgemeinen Formel I können entweder als Basen oder in Form ihrer Salze aus den Reaktionsgemischen isoliert werden. Sie lassen sich als Basen mit geeigneten anorganischen oder organischen Säuren nach bekannten Verfahren in die Salze überführen.

Bevorzugt werden physiologisch verträgliche Salze der Verbindung der Formel I. Hierfür sind als anorganische Säuren beispielsweise Halogenwasserstoffsäuren, zum Beispiel Salzsäure oder Schwefelsäure und als organische Säuren zum Beispiel Fumarsäure, Maleinsäure, Zitronensäure und Weinsäure geeignet. Zur Herstellung wird die heiße alkoholische Lösung der Base mit der alkoholischen Lösung einer geeigneten Säure versetzt und man erhält nach Etherzusatz das Salz.

Die vorliegenden Verbindungen der Formel I können aufgrund eines oder zweier Chiralitätszentren als Racemate oder als Gemisch von zwei Diastereoisomeren vorliegen. Diastereoisomere können in bekannter Weise aufgrund der physikalisch-chemischen Unterschiede getrennt werden. Racemate können nach bekannten Methoden getrennt werden, beispielsweise durch Umkristallisieren in optisch aktiven Lösungsmitteln, durch Mikroorganismen oder Reaktion mit einer optisch aktiven Säure, die mit der racemischen Verbindung ein Salz bildet, Trennung der Diastereoisomere durch fraktionierte Kristallisation und Freisetzung der Enantiomeren durch geeignete Mittel. Besonders geeignete optisch aktive Säuren sind beispielsweise die d- und l-Formen der Weinsäure, Diorthotoloylweinsäure, Äpfelsäure, Mandelsäure, Kamphersulfonsäure oder Chininsäure. Vorteilhafterweise wird der aktivere der Antipoden isoliert. Gemäß der Erfindung ist es jedoch auch möglich, die reinen Enantiomeren durch asymmetrische Synthese zu erhalten.

Die folgenden Beispiele dienen zur Erläuterung der Erfindung:

## Beispiel 1

2-[2-[2-Hydroxy-3-(3-(4-hydroxyphenyl)-1-methyl-propylamino)-propoxy]-4-chlor-phenyl]-1,3,4-oxadiazol

5,1 g (0.02 Mol) 2-[2-(2,3-Epoxypropoxy)-4-chlor-phenyl]-1,3,4-oxadiazol und 3,3 g (0,02 Mol) 4-(4-Hydroxyphenyl) —butylamin-2 werden in 100 ml t-Butanol 4 Stunden bei 80°C gerührt. Nach Entfernen des Lösungsmittels wird der Rückstand durch Chromatografie an Kieselgel gereinigt ($CH_2Cl_2/CH_3OH=3/2$). Man erhält nach Fraktionierung 4 g eines farblosen Öls, dessen Struktur 2-[2-[2-Hydroxy-3-(3-(4-hydroxyphenyl)-1-methyl-propylamino)-propoxy]-4-chlor-phenyl]-1,3,4-oxadiazol entspricht.

Zur Herstellung des Oxalats löst man 3,3 g der Base in heißem Ethanol und versetzt mit 0,36 g Oxalsäure. Nach Erkalten wird der kristalline Niederschlag abgesaugt und mit Ethanol und Äther gewaschen. Man erhält 3,3 g des 2-[2-[2-Hydroxy-3-(3-(4-hydroxyphenyl)-1-methyl-propylamino)-propoxy]-4-chlor-phenyl]-1,3,4-oxadiazol-oxalats. Fp. 194 - 196°C.

Analog Beispiel 1 wurden die folgenden Verbindungen der allgemeinen Formel I hergestellt (X= - bedeutet eine Einfachbindung):

| Beispiel | $R^1$ | $R^2$ | $R^3$ | $R^4$ | X | n | Salz | Fp. °C |
|---|---|---|---|---|---|---|---|---|
| 2 | 4-Cl | $CH_3$ | H | 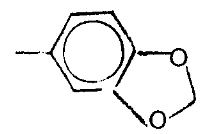 | - | 2 | Oxalat | 159-160 |
| 3 | 4-Cl | $CH_3$ | H | 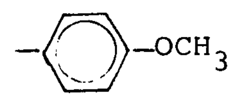 | - | 2 | Oxalat | 187-190 |
| 4 | 4-Cl | $CH_3$ | $CH_3$ | 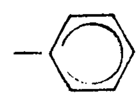 | - | 2 | HCl | 184-186 |
| 5 | 4-Cl | $CH_3$ | $CH_3$ | 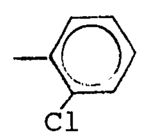 | - | 2 | HCl | 175-177 |

| Beispiel | $R^1$ | $R^2$ | $R^3$ | $R^4$ | X | n | Salz | Fp. °C |
|---|---|---|---|---|---|---|---|---|
| 6 | 4-Cl | $CH_3$ | $CH_3$ | 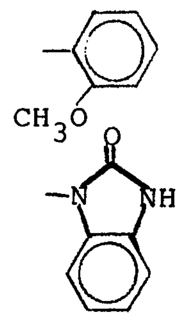 | - | 2 | Fumarat | 203-205 |
| 7 | 4-Cl | $CH_3$ | $CH_3$ |  | - | 1 | Fumarat | 222-225 |
| 8 | 4-Cl | $CH_3$ | $CH_3$ | 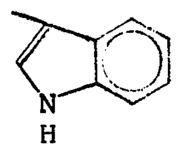 | - | 1 | Fumarat | 208-211 |
| 9 | 4-Cl | H | $CH_3$ | 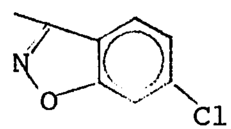 | - | 2 | Fumarat | 174-176 |
| 10 | 4-Cl | H | H | 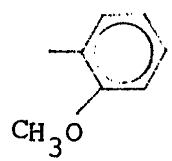 | O | 1 | Oxalat | 144-147 |

| Beispiel | $R^1$ | $R^2$ | $R^3$ | $R^4$ | X | n | Salz | Fp. °C |
|---|---|---|---|---|---|---|---|---|
| 11 | 4-Cl | H | H | 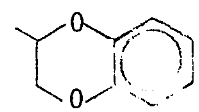 | - | O | Fumarat | amorph |
| 12 | 5-n-$C_6H_{13}$CONH | H | H | 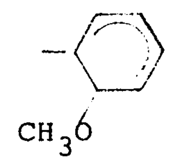 | O | 1 | Maleinat | 133-135 |
| 13 | H | H | H | 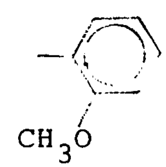 | O | 1 | Oxalat | 121-122 |
| 14 | 4-Cl | H | H | 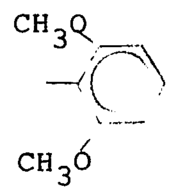 | O | 1 | Oxalat | 100-105 |

| Beispiel | $R^1$ | $R^2$ | $R^3$ | $R^4$ | X | n | Salz | Fp. °C |
|---|---|---|---|---|---|---|---|---|
| 15 | 4-Cl | H | H | 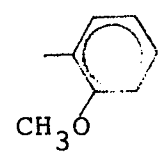 | - | 1 | Oxalat | 158-160 |
| 16 | 4-Cl | $CH_3$ | $CH_3$ | 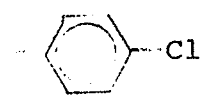 | - | 2 | HCl | 137-139 |
| 17 | 4-Cl | $CH_3$ | $CH_3$ | 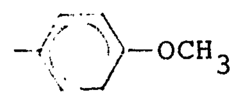 | - | 2 | HCl | 202-204 |
| 18 | 4-Cl | $CH_3$ | $CH_3$ | 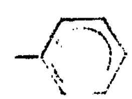 | - | 3 | HCl | 135-137 |

| Beispiel | $R^1$ | $R^2$ | $R^3$ | $R^4$ | X | n | Salz | Fp. °C |
|---|---|---|---|---|---|---|---|---|
| 19 | 4-Cl | $CH_3$ | $CH_3$ | 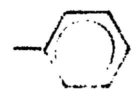 | - | 1 | | |
| 20 | 4-Cl | $CH_3$ | $CH_3$ | 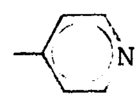 | - | 2 | HCl | 235-237 |
| 21 | 4-Cl | $CH_3$ | $CH_3$ | 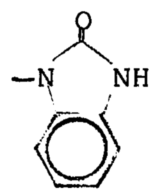 | - | 3 | HCl | amorph |
| 22 | 4-Cl | $CH_3$ | H | 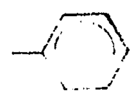 | - | 2 | Oxalat | 166-168 |

| Beispiel | $R^1$ | $R^2$ | $R^3$ | $R^4$ | X | n | Salz | Fp.°C |
|---|---|---|---|---|---|---|---|---|
| 23 | 4-Cl | $CH_3$ | H | 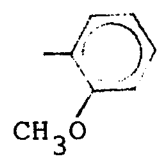 | O | 1 | | |
| 24 | 4-Cl | $CH_3$ | H | 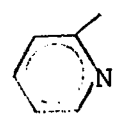 | – | 2 | | |

| Beispiel | $R^1$ | $R^2$ | $R^3$ | $R^4$ | X | n | Salz | Fp.°C |
|---|---|---|---|---|---|---|---|---|
| 25 | 4-Cl | $CH_3$ | $CH_3$ | 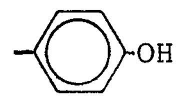 | - | 2 | HCl | 224-226 |
| 26 | 4-Cl | $CH_3$ | $CH_3$ | 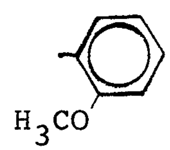 | - | 3 | HCl | 154-156 |
| 27 | 4-Cl | $CH_3$ | $CH_3$ | 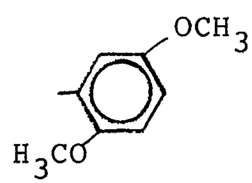 | - | 2 | HCl | 172-174 |
| 28 | 4-Cl | $CH_3$ | $CH_3$ | 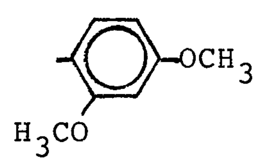 | - | 2 | HCl | 166-168 |

| Beispiel | $R^1$ | $R^2$ | $R^3$ | $R^4$ | X | n | Salz | Fp.°C |
|---|---|---|---|---|---|---|---|---|
| 29 | 4-Cl | $CH_3$ | $CH_3$ | 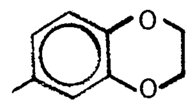 | - | 2 | HCl | 195-197 |
| 30 | 4-Cl | $CH_3$ | $CH_3$ | 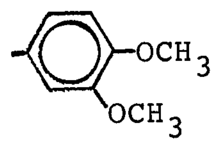 | - | 2 | HCl | 170-173 |
| 31 | 4-Cl | $CH_3$ | $CH_3$ | 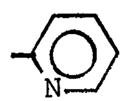 | - | 2 | HCl | 203-207 |
| 32 | 4-Cl | $CH_3$ | H | 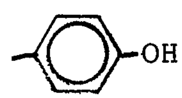 | - | 2 | Oxalat | 194-196 |

| Beispiel | $R^1$ | $R^2$ | $R^3$ | $R^4$ | X | n | Salz | Fp.°C |
|---|---|---|---|---|---|---|---|---|
| 33 | 4-Cl | $CH_3$ | H | 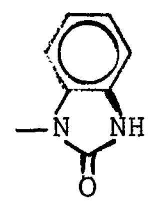 | - | 1 | Fumarat | 200-205 |
| 34 | 4-Cl | H | H | 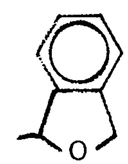 | - | 0 | Base | 136-141 |
| 35 | 4-Cl | H | H | 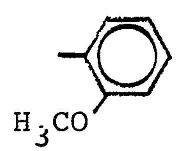 | - | 1 | Oxalat | 150-160 |

## Beispiel 36

### Herstellung von Tabletten

Tabletten, welche die nachstehend angegebenen Bestandteile enthalten, werden nach bekannten Arbeitsweisen hergestellt. Diese sind für die Behandlung von den vorstehend genannten Krankheiten, insbesondere Hypertonie, in einer Dosierungsmenge von 40 mg einmal bis zweimal täglich geeignet.

| | Tablette A | Tablette B |
|---|---|---|
| 2/2-/2-Hydroxy-3-(3-(4-hydroxyphenyl)-1-methyl-propylamino)-propoxy7-4-chlor-phenyl7-1,3,4-oxadiazol | 40 mg | 20 mg |
| Lactose | 90 mg | 90 mg |
| Maisstärke | 5 mg | 5 mg |
| Magnesiumstearat | 1 mg | 1 mg |

## Beispiel 37

### Herstellung von Ampullen

Ampullen, die die im folgenden genannten Bestandteile enthalten, lassen sich in bekannter Weise herstellen. Wirkstoff und Natriumchlorid werden in Wasser gelöst und unter Stickstoff in Glasampullen abgefüllt.

| | |
|---|---|
| Hydrochlorid des 2-/2-/2-Hydroxy-3-(3-(4-hydroxyphenyl)-1-methyl-propylamino)-propoxy7-4-chlor-phenyl7-1,3,4-oxadiazols | 10 mg |
| Natriumchlorid | 18 mg |
| dest. Wasser ad | 2,0 ml |

## Patentansprüche

1. Substituierte Phenoxyalkanolaminopropanole der allgemeinen Formel I

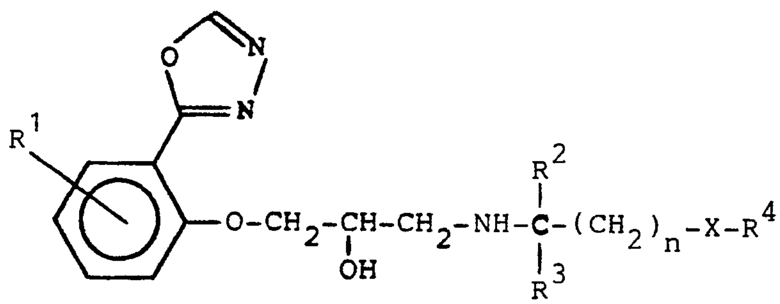

(I)

in der

$R^1$ ein Wasserstoff- oder Halogenatom, eine Alkyl- oder Alkoxygruppe mit jeweils 1 bis 4 Kohlenstoffatomen, eine Aminogruppe oder Acylaminogruppe,

$R^2$ und $R^3$, die gleich oder verschieden sein können, ein Wasserstoffatom oder eine Alkylgruppe mit 1 bis 3 Kohlenstoffatomen

$R^4$ eine Phenylgruppe oder eine substituierte Phenylgruppe, die durch ein Halogenatom, eine oder zwei Alkoxygruppen, eine Methylendioxy-Gruppe, eine Hydroxy-Gruppe substituiert sein kann, eine Pyridylgruppe, eine Indolyl-Gruppe, eine gegebenenfalls durch ein Halogenatom substituierte 1,2-Benzisoxazolylgruppe, den Benzimidazol-2-on-, 1,4-Benzodioxan- oder Benzoxolan-Rest,

X ein Sauerstoffatom oder eine Einfachbindung und

n die Zahlen 0, 1, 2 oder 3 bedeuten,

sowie ihre Säureadditionssalze.

2. Verfahren zur Herstellung von Verbindungen der Formel I gemäß Anspruch 1, dadurch gekennzeichnet, daß man Verbindungen der allgemeinen Formel IIa oder IIb oder deren Gemische,

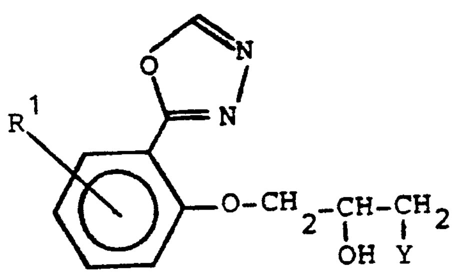

(IIa)

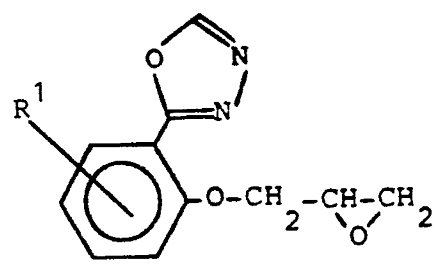

(IIb)

wobei Y für ein Halogenatom steht, mit einem Amin der allgemeinen Formel III

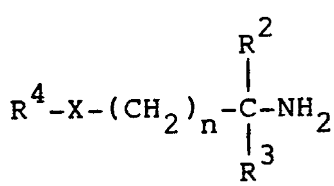

(III)

in der $R^2$, $R^3$, $R^4$, X und n die oben angegebene Bedeutung haben, umsetzt.

3. Pharmazeutisches Präparat, dadurch gekennzeichnet, daß es eine oder mehrere der Verbindungen gemäß Anspruch 1 oder deren physiologisch verträgliche Salze und gegebenenfalls übliche Trägerstoffe und/oder Verdünnungsmittel enthält.

4. Verbindungen der Formel I gemäß Anspruch 1 zur Anwendung als therapeutische Wirkstoffe, insbesondere zur Behandlung von Hypertonie, Durchblutungsstörungen, peripheren Durchblutungsstörungen, Angina pectoris und Coronarinsuffizienz.